# EUROPÄISCHE PATENTSCHRIFT

(11) **EP 2 982 384 B1**
(45) Veröffentlichungstag und Bekanntmachung des Hinweises auf die Patenterteilung: **26.07.2017**
(21) Anmeldenummer: 15168461.0
(22) Anmeldetag: 20.05.2015
(51) Int. Cl.: A61L 2/18, A01N 25/34, A47L 13/17, C11D 17/04, B65D 65/14

(54) **DESINFEKTIONSTUCH MIT FARBINDIKATION**
DISINFECTION WIPE WITH COLOUR INDICATION
LINGUETTE DESINFECTANTE COMPRENANT UN INDICATEUR DE COULEUR

(30) Priorität: 05.08.2014 DE 202014103637 U
(43) Veröffentlichungstag der Anmeldung: 10.02.2016
(73) Patentinhaber: Wilbert, Thomas, 80805 München (DE)
(72) Erfinder: Wilbert, Thomas, 80805 München (DE)
(74) Vertreter: 2s | ip Schramm Schneider Patentanwälte Rechtsanwälte

(56) Entgegenhaltungen:
- EP-A1- 1 295 613
- WO-A1-2005/011756
- WO-A1-2012/152676
- WO-A1-2014/029462
- WO-A1-2015/040366
- WO-A2-2013/022964
- DE-C- 896 928
- GB-A- 2 404 337
- US-A1- 2006 051 266

## Beschreibung

### Gegenstand der Erfindung

Die Erfindung betrifft ein Desinfektionstuch.

### Hintergrund der Erfindung

Tuch- bzw. Feuchttuchspender sind aus dem Stand der Technik allgemein bekannt. Im medizinischen Bereich bzw. in sanitären Einrichtungen, etwa in Krankenhäusern, Arztpraxen oder Zahnarztpraxen ist es erforderlich, Ablageflächen und/oder Hilfsmittel regelmäßig zu reinigen und zu desinfizieren. Die hierfür verwendeten Desinfektionstücher werden dabei einem Tuchspender entnommen. Ein Tuchspender kann beispielsweise einen Spendereimer umfassen, der im Deckel eine Entnahmeöffnung aufweist, durch die das Desinfektionstuch herausgezogen werden kann.

Wenn sich kein Desinfektionstuch mehr in dem Tuchspender bzw. Spendereimer befindet, werden diese regelmäßig einer Aufbereitung zugeführt, bei der der Tuchspender bzw. Spendereimer gereinigt und desinfiziert wird, bevor neue Desinfektionstücher in den Tuchspender bzw. Spendereimer gegeben werden. Hierzu ist es bekannt, den Spendereimer zunächst mit heißem Wasser auszuspülen und anschließend zu trocknen bzw. trocknen zu lassen. Anschließend wird der Spendereimer desinfiziert und nochmals getrocknet. Zum Desinfizieren und abschließenden Trocknen können speziell hierzu vorgesehene Tücher verwendet werden.

Diese Vorgehensweise ist einerseits zeitaufwändig, weil der Trocknungsvorgang eine gewisse Zeit in Anspruch nimmt und der nächste Aufbereitungsschritt erst durchgeführt werden kann, wenn der Trocknungsvorgang abgeschlossen ist. Andererseits hat sich diese Vorgehensweise auch deshalb als nachteilig herausgestellt, weil die für die Aufbereitung vorgesehenen Tücher in vielen Fällen in der falschen Reihenfolge benutzt werden, sodass das gewünschte Aufbereitungs- bzw. Reinigungsergebnis nicht sichergestellt werden kann. Ein weiterer Nachteil besteht darin, dass das zum heißen Ausspülen des Behältnisses verwendete Wasser gegebenenfalls mit noch in dem Behältnis vorhandenen Desinfektionsmitteln kontaminiert werden kann und daher einer gesonderten Entsorgung bzw. einer Aufbereitung zugeführt werden muss, was mit zusätzlichen Kosten verbunden ist.

Aus der WO 2005/011756 A1, der GB 2404337 A, der US 2006/0051266 A1 und aus der WO 2014/040366 A1 sind jeweils ein Reinigungssystem bekannt, das eine Box aufweist, in der eine Anzahl von Reinigungstücher angeordnet sind. Die Reinigungstücher sind jeweils in Tütchen verpackt, die mit einer Schrift und einem Muster bedruckt sind.

Aus der WO 2014/029462 A1 ist ein Verfahren zur Herstellung eines Gebindes aus mehreren Behältern bekannt, wobei das Gebinde mit einer Schrumpffolie zusammengehalten wird, wobei vor dem Anbringen der Schrumpffolie eine Banderole angeordnet ist, und wobei die Schrumpffolie an Stirnseiten des Gebindes verklebt wird.

Aus der DE896928 C ist eine Umhüllung aus einem reißbaren Stoff, etwa für Teeaufgussbeutel, bekannt, wobei ein Etikett des umhüllten Gegenstandes einen lostrennbaren Teil der Umhüllung bildet.

Aus der WO 2012/152676 A1 ist eine Verpackung für streifenförmige Gegenstände, insbesondere Kaugummistreifen, bekannt.

Aus der WO 2013/022964 A2 ist eine Verpackung bekannt, die mehrere Kammern aufweist, wobei die Verpackung mit einer abziehbaren Lasche verschlossen ist, wobei beim Abziehen der Lasche die Kammern in einer vorbestimmten Reihenfolge freigegeben bzw. geöffnet werden.

### Aufgabe der Erfindung

Der Erfindung liegt daher die Aufgabe zugrunde, Lösungen für ein Aufbereiten bzw. Desinfizieren von wiederverwendbaren Tuchspendern bzw. Spendereimern für Desinfektionstücher bereitzustellen, die die aus dem Stand der Technik bekannten Nachteile zumindest teilweise vermeiden und mit denen einerseits der zeitliche Aufwand für ein Wiederaufbereiten erheblich reduziert werden kann und andererseits das gewünschte Reinigungs- bzw. Aufbereitungsergebnis sicher gewährleistet werden kann, ohne dass hierbei die Umwelt zusätzlich belastet wird.

### Erfindungsgemäße Lösung

Diese Aufgabe wird erfindungsgemäß mit einem Desinfektionstuch nach Anspruch 1 gelöst. Vorteilhafte Ausgestaltungen und Weiterbildungen der Erfindung sind in den jeweiligen abhängigen Ansprüchen angegeben.

Bereitgestellt wird demnach ein Desinfektionstuch, insbesondere zur Flächendesinfektion in sanitären Einrichtungen, wobei
- das Desinfektionstuch vorgesehen ist, in ein Behältnis, insbesondere Tuchspender, eingebracht zu werden, wobei das Behältnis vor Einbringen des Desinfektionstuches einer Reinigung bzw. Desinfektion zu unterziehen ist,
- das Desinfektionstuch in einer Schutzverpackung verpackt ist,
- das Desinfektionstuch vorzugsweise ohne die Schutzverpackung in das Behältnis einbringbar ist, und
- an der Schutzverpackung ein Reinigungssystem angeordnet ist, zum Reinigen und/oder Desinfizieren des Behältnisses, wobei das Reinigungssystem zumindest ein erstes verpacktes Reinigungstuch, ein zweites verpacktes Reinigungstuch und ein drittes verpacktes Reinigungstuch umfasst, wobei die Verpackungen der Reinigungstücher jeweils eine unterschiedliche Farbmarkierung aufweisen.

Die Farbmarkierungen können indikativ dafür sein, in welcher Reihenfolge die Reinigungstücher zum Reinigen bzw. Desinfizieren des Behältnisses anzuwenden sind. Damit wird sichergestellt, dass die Reinigungstücher in einer bestimmten Reihenfolge angewandt werden, sodass auch gewährleistet ist, dass der gewünschte Reinigungs- bzw. Aufbereitungserfolg erzielt wird. Durch Anwenden der drei Reinigungstücher in der durch die Farbmarkierung vorgegebenen Reihenfolge kann auf ein heißes Ausspülen des Behältnisses verzichtet werden. Ein Aufbereiten / gesondertes Entsorgen von Spülwasser wird damit vermieden. Ferner werden die Trocknungszeiten erheblich verringert, sodass der zeitliche Aufwand für das Aufbereiten eines Behältnisses erheblich verringert wird.

Vorteilhaft ist es, wenn die Reinigungstücher mit einem Befestigungsmittel lösbar an der Schutzverpackung des Desinfektionstuches befestigt sind. Damit kann sichergestellt werden, dass vor dem Einbringen des Desinfektionstuches in das Behältnis das Behältnis mit den Reinigungstüchern aufbereitet wird.

Vorteilhaft ist hierbei, wenn das Befestigungsmittel angepasst ist, beim Lösen des Befestigungsmittels von der Schutzverpackung des Desinfektionstuches die Reinigungstücher in einer vorbestimmten Reihenfolge zur Verwendung freizugeben. Das bedeutet, dass beim Lösen des Befestigungsmittels von der Schutzverpackung zunächst jenes Reinigungstuch zur Verwendung freigegeben wird, welches für den gewünschten Reinigungs- bzw. Aufbereitungserfolg als erstes verwendet werden muss. Anschließend wird dementsprechend das zweite zu verwendende Reinigungstuch freigegeben und abschließend wird das dritte zu verwendende Reinigungstuch freigegeben.

Vorteilhaft ist demnach also, dass in der vorbestimmten Reihenfolge zuerst das erste verpackte Reinigungstuch, anschließend das zweite verpackte Reinigungstuch und abschließend das dritte verpackte Reinigungstuch freigegeben werden.

In einer Ausgestaltung der Erfindung kann das Befestigungsmittel ein Befestigungsband, insbesondere ein Klebeband umfassen, wobei die beiden Enden des Befestigungsbandes an der Schutzverpackung des Desinfektionstuches befestigt sind und wobei zwischen der Schutzverpackung und dem Befestigungsband die Reinigungstücher angeordnet sind.

Als vorteilhaft hat sich herausgestellt, wenn das Befestigungsband an einem ersten Endabschnitt und an einem zweiten Endabschnitt mit der Schutzverpackung verbunden, vorzugsweise verklebt ist, wobei die Reinigungstücher zwischen den beiden Endabschnitten angeordnet sind.

In einer Ausgestaltung der Erfindung kann die Schutzverpackung des Desinfektionstuches eine Sollbruchstelle aufweisen, entlang der die Schutzverpackung zur Entnahme des Desinfektionstuches geöffnet werden kann. Die Sollbruchstelle kann beispielsweise als Perforation ausgestaltet sein.

Vorteilhaft ist hierbei, wenn das Befestigungsband so an der Schutzverpackung angeordnet ist, dass die Sollbruchstelle zumindest teilweise von dem Befestigungsband abgedeckt ist. Insbesondere kann das Befestigungsband so an der Schutzverpackung angeordnet sein, dass das Befestigungsband die Sollbruchstelle kreuzt. Damit ist sichergestellt, dass die Schutzverpackung erst dann zur Entnahme des Desinfektionstuches geöffnet werden kann, nachdem das Befestigungsband und damit die Reinigungstücher von der Schutzverpackung abgenommen wurden und das Behältnis gereinigt wurde.

Besonders vorteilhaft ist es, wenn das Befestigungsband im Bereich des ersten Endabschnittes eine Perforation aufweist, um das Befestigungsband zur Freigabe der Reinigungstücher zu durchtrennen. Das Befestigungsband ist vorteilhafterweise so ausgestaltet, dass dieses ohne weitere Hilfsmittel, beispielsweise Schere, nur an der Perforation durchtrennt werden kann, sodass in jedem Fall sichergestellt ist, dass die Reinigungstücher in der vorbestimmten Reihenfolge zur Verwendung freigegeben werden.

In einer Ausgestaltung der Erfindung kann das Befestigungsband in den Bereichen zwischen jeweils zwei Reinigungstüchern eine weitere Perforation aufweisen. Damit kann jedes Reinigungstuch zusammen mit dem entsprechenden Abschnitt des Befestigungsbandes von der Schutzverpackung gelöst werden, was eine einfachere Handhabung ermöglicht.

In einer Ausgestaltung der Erfindung kann das Befestigungsband ein erstes Befestigungsband, ein zweites Befestigungsband und ein drittes Befestigungsband umfassen, wobei mit jedem Befestigungsband jeweils ein Reinigungstuch lösbar an der Schutzverpackung des Desinfektionstuches befestigt ist, derart, dass die Perforation an dem ersten Endabschnitt des zweiten und/oder dritten Befestigungsbandes erst dann freigegeben ist, wenn das erste Befestigungsband von der Schutzverpackung gelöst ist. Auch damit wird gewährleistet, dass die an der Schutzverpackung angebrachten Reinigungstücher nur in der für das gewünschte Reinigungs- bzw. Aufbereitungsergebnis vorgesehenen Reihenfolge von der Schutzverpackung gelöst werden können und damit in der vorgesehenen Reihenfolge angewandt werden. Das bedeutet, dass die Perforation des zweiten Befestigungsbandes erst dann durchtrennt werden kann, wenn das erste Reinigungstuch von der Schutzverpackung abgenommen wurde.

Vorteilhaft ist hierbei, wenn der erste Endabschnitt des zweiten und/oder dritten Befestigungsbandes zwischen der Schutzverpackung und dem ersten verpackten Reinigungstuch und/oder dem zweiten verpackten Reinigungstuch angeordnet ist.

Das Befestigungsband kann zumindest in den Bereichen der Reinigungstücher und an der den Reinigungstüchern zugewandten Oberfläche eine Klebeschicht aufweisen. Damit kann gewährleistet werden, dass sich die Reinigungstücher nach Durchtrennen der Perforation an dem ersten Endabschnitt des Befestigungsbandes selbstständig lösen, z.B. weg- oder herunterfallen, weil die Reinigungstücher aufgrund der Klebeschicht an dem Befestigungsband haften bleiben.

Als besonders vorteilhaft hat sich herausgestellt, wenn die Verpackung des ersten Reinigungstuches eine erste, rote Farbmarkierung, die Verpackung des zweiten Reinigungstuches eine zweite, gelbe Farbmarkierung und die Verpackung des dritten Reinigungstuches eine dritte, grüne Farbmarkierung aufweisen. Damit wird für die Reihenfolge der Verwendung der Reinigungstücher ein Ampelsystem zur Verfügung gestellt, wonach zuerst das rote Reinigungstuch und als letztes das grüne Reinigungstuch anzuwenden ist.

In einer Ausgestaltung der Erfindung kann die Schutzverpackung des Desinfektionstuches eine Kunststofffolie aufweisen.

Das Desinfektionstuch kann eine Anzahl von Desinfektionstüchern und/oder ein Desinfektionstuch mit vorzugsweise gleich beabstandeten Perforationen umfassen.

Das Reinigungstuch, dessen Verpackung eine rote Farbmarkierung aufweist, kann kationische Tenside, z.B. quartäre Ammoniumverbindungen, aufweisen, sodass dieses Tuch zur Reinigung des Behältnisses, des Deckels des Behältnisses und ggf. der Entnahmevorrichtung verwendet werden kann. Zudem können diesem Reinigungstuch Duftstoffe beigegeben werden.

Das Reinigungstuch, dessen Verpackung eine gelbe Farbmarkierung aufweist, wird zum Trocknen der in dem ersten Schritt gereinigten Flächen bzw. Oberflächen verwendet.

Das Reinigungstuch, dessen Verpackung eine grüne Farbmarkierung aufweist, kann Ethanol, Didecyl-dimethyl-ammoniumchlorid, Duft- und sonstige Hilfsstoffe aufweisen und wird zur abschließenden Desinfektion des Behältnisses, des Deckels des Behältnisses und ggf. der Entnahmevorrichtung verwendet. Durch diese Zusammensetzung kann das Spendersystem bereits nach etwa 1 Minute mit neuen Desinfektionstüchern befüllt werden. Ein abschließendes Trocknen des Spendersystems ist nicht mehr notwendig.

Zum besseren Verständnis der Erfindung wird des Weiteren ein Reinigungssystem zum Reinigen und/oder Desinfizieren von Behältnissen eines Tuchspenders, insbesondere Feuchttuchspender, beschrieben, wobei das Reinigungssystem zumindest ein erstes verpacktes Reinigungstuch, ein zweites verpacktes Reinigungstuch und ein drittes verpacktes Reinigungstuch umfasst, wobei die Verpackungen der Reinigungstücher jeweils eine unterschiedliche Farbmarkierung aufweisen.

Vorteilhaft ist es, wenn die Verpackung des ersten Reinigungstuches eine erste, rote Farbmarkierung, die Verpackung des zweiten Reinigungstuches eine zweite, gelbe Farbmarkierung und die Verpackung des dritten Reinigungstuches eine dritte, grüne Farbmarkierung aufweisen. Damit kann sichergestellt werden, dass die Reinigungstücher entsprechend der Farbmarkierungen, die ein Ampelsystem bilden, in der richtigen Reihenfolge angewandt werden.

Die Farbmarkierungen der Verpackungen der Reinigungstücher geben die Reihenfolge an, in der die Reinigungstücher beim Reinigen und/oder Desinfizieren des Behältnisses anzuwenden sind, wobei das erste Reinigungstuch, dessen Verpackung die rote Farbmarkierung aufweist, vor dem zweiten Reinigungstuch, dessen Verpackung die gelbe Farbmarkierung aufweist, anzuwenden ist und wobei das zweite Reinigungstuch, dessen Verpackung die gelbe Farbmarkierung aufweist, vor dem dritten Reinigungstuch, dessen Verpackung die grüne Farbmarkierung aufweist, anzuwenden ist.

Das Reinigungssystem kann ein Befestigungsmittel umfassen, mit dem die verpackten Reinigungstücher an einer Schutzverpackung eines Desinfektionstuches lösbar befestigbar sind.

Das Befestigungsmittel kann ein Befestigungsband, insbesondere Klebeband, umfassen.

Vorteilhaft ist es, wenn ein erster Endabschnitt und ein zweiter Endabschnitt des Befestigungsmittels an der Schutzverpackung des Desinfektionstuches befestigbar sind.

Weiter vorteilhaft ist es, wenn das Befestigungsmittel im Bereich seines ersten Endabschnittes eine Perforation aufweist.

In einer weiteren Ausgestaltung kann das Befestigungsmittel zumindest zwei weitere Perforationen aufweisen, die zwischen dem ersten Endabschnitt und dem zweiten Endabschnitt angeordnet sind.

In einer Ausgestaltung kann das Befestigungsmittel des Reinigungssystems zumindest drei Befestigungsbänder, insbesondere Klebebänder, umfassen, wobei mit jedem der drei Befestigungsbänder jeweils ein Reinigungstuch an der Schutzverpackung des Desinfektionstuches lösbar befestigbar ist.

Die verpackten Reinigungstücher können derart mit dem Befestigungsband bzw. mit den Befestigungsbändern an der Schutzverpackung des Desinfektionstuches lösbar befestigbar sein, dass sie beim Lösen des Befestigungsbandes bzw. der Befestigungsbänder von der Schutzverpackung in einer vorbestimmten Reihenfolge zur Verwendung freigegeben werden.

Beschrieben wird ferner ein Befestigungsmittel zum lösbaren Befestigen eines Reinigungssystems, das zumindest ein erstes verpacktes Reinigungstuch, ein zweites verpacktes Reinigungstuch und ein drittes verpacktes Reinigungstuch umfasst, an einer Schutzverpackung eines Desinfektionstuches bereitgestellt, wobei das Befestigungsmittel angepasst ist, beim Lösen des Befestigungsmittels von der Schutzverpackung die an der Schutzverpackung mit dem Befestigungsmittel befestigten Reinigungstücher in einer vorbestimmten Reihenfolge zur Verwendung freizugeben.

Das Befestigungsmittel kann zumindest ein Befestigungsband, insbesondere Klebeband, umfassen.

Vorteilhaft ist es, wenn das Befestigungsmittel einen ersten Endabschnitt und einen zweiten Endabschnitt aufweist, mit denen das Befestigungsmittel an der Schutzverpackung, vorzugsweise durch eine Klebeverbindung, befestigbar ist.

Weiter vorteilhaft ist es, wenn das Befestigungsmittel an dem ersten Endabschnitt, d.h. im Bereich des ersten Endabschnittes, eine Perforation aufweist.

In einer Ausgestaltung kann das Befestigungsmittel zumindest zwei weitere Perforationen aufweisen, die zwischen dem ersten Endabschnitt und dem zweiten Endabschnitt angeordnet sind.

Als vorteilhaft hat es sich herausgestellt, wenn das Befestigungsmittel zwischen den Perforationen und zwischen dem zweiten Endabschnitt und der dazu benachbarten Perforation klebende Abschnitte aufweist, die beim Befestigen der Reinigungstücher an der Schutzverpackung des Desinfektionstuches den Reinigungstüchem zugewandt sind, sodass die Reinigungstücher an dem Befestigungsmittel haften bleiben, wenn das Befestigungsmittel von der Schutzverpackung gelöst bzw. abgenommen wird.

Mit dem Reinigungssystem bzw. mit dem erfindungsgemäßen Desinfektionstuch, an dem ein Reinigungssystem angeordnet ist, sowie mit dem Befestigungsmittel werden die für eine Desinfektion bzw. Aufbereitung von Behältnissen für Desinfektionstücher notwendigen Reinigungstücher so an das neu in das Behältnis einzubringende Desinfektionstuch angeordnet bzw. befestigt, dass weitgehend gewährleistet ist, dass die Reinigungstücher nur in einer vorbestimmten Reihenfolge verwendet werden können. Zudem ist ein heißes Ausspülen des Behältnisses nicht mehr notwendig. Des Weiteren wird der gesamte zeitliche Aufwand für die Aufbereitung der Behältnisse erheblich verringert.

### Kurzbeschreibung der Figuren

Die Erfindung wird im Folgenden anhand einer schematischen Zeichnung näher erläutert. Es zeigen:
- Fig. 1: ein Desinfektionstuch mit einem daran angeordneten Reinigungssystem; und
- Fig. 2: drei unterschiedliche Ausgestaltungen eines Reinigungssystems, das an einer Schutzverpackung eines Desinfektionstuches angeordnet ist, in einem Querschnitt.

### Detaillierte Beschreibung der Erfindung

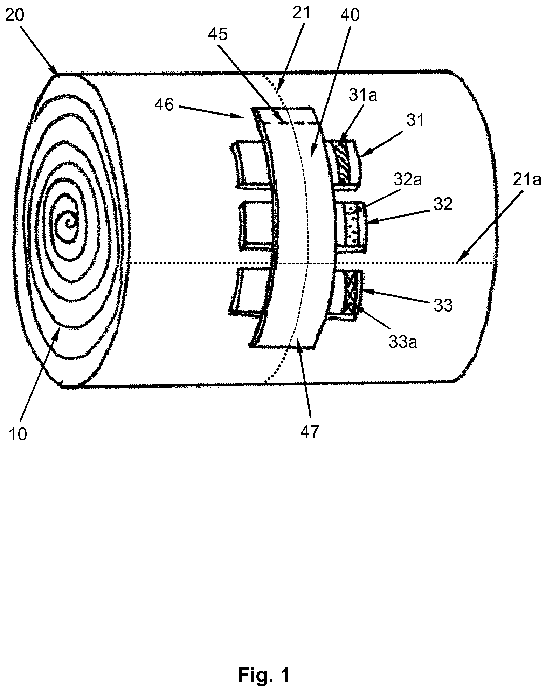

Fig. 1 zeigt ein Desinfektionstuch 10, das insbesondere zur Flächendesinfektion in sanitären Einrichtungen, etwa in Krankenhäusern, Arztpraxen oder Zahnarztpraxen verwendet werden kann.

Das Desinfektionstuch 10 kann aus einem Vliesstoff hergestellt sein. Andere Materialien sind aber möglich. Ferner ist das Desinfektionstuch 10 in einer Schutzverpackung 20, etwa eine Folie, verpackt, um eine Kontamination während des Transportes zu verhindern. Am Ort der Verwendung wird das Desinfektionstuch 10 aus der Schutzverpackung 20 herausgenommen und in einen Tuchspender, etwa Spendereimer, eingebracht, wobei dem Desinfektionstuch 10 in dem Spendereimer zusätzlich ein flüssiges Desinfektionsmittel zugegeben werden kann. In einer Ausgestaltung des Desinfektionstuches 10 kann dieses bereits mit der gewünschten Desinfektionslösung getränkt bzw. benetzt sein, sodass kein zusätzliches Desinfektionsmittel in den Spendereimer gegeben werden muss. Der Spendereimer wird dann verschlossen, wobei ein Ende des Desinfektionstuches an einer hierfür vorgesehenen Öffnung des Spendereimers nach außen geführt wird.

Bei dem in Fig. 1 gezeigten Desinfektionstuch 10 handelt es sich um sogenanntes Nachfüll-Desinfektionstuch, mit welchem ein Tuchspender bzw. ein Spendereimer wieder befüllt werden kann, sodass die Tuchspender bzw. Spendereimer wiederverwendet werden können. Die Tuchspender bzw. Spendereimer dürfen allerdings nur dann wiederverwendet werden, wenn sie vor Einbringen des Desinfektionstuches und gegebenenfalls der Desinfektionslösung einer Aufbereitung zugeführt wurden, bei der das Behältnis gereinigt und desinfiziert wird. Die Reinigung bzw. Desinfektion des Behältnisses muss einem bestimmten Ablauf folgen, um das gewünschte Aufbereitungsergebnis zu erzielen. Insbesondere ist es notwendig, dass bestimmte Reinigungs- bzw. Desinfektionsschritte in einer vorbestimmten Reihenfolge durchgeführt werden.

Für das Aufbereiten der Behältnisse wird ein Reinigungssystem zur Verfügung gestellt, welches ein erstes verpacktes Reinigungstuch 31, ein zweites verpacktes Reinigungstuch 32 und ein drittes verpacktes Reinigungstuch 33 umfasst. Die Verpackungen der Reinigungstücher 31, 32, 33 weisen jeweils unterschiedliche Farbmarkierungen auf, wobei die Verpackung des ersten Reinigungstuches 31 eine rote Farbmarkierung 31 a, die Verpackung des zweiten Reinigungstuches 32 eine gelbe Farbmarkierung 32a und die Verpackung des dritten Reinigungstuches 33 eine grüne Farbmarkierung 33a aufweisen.

Die Reinigungstücher 31, 32, 33 sind vorgesehen, das Behältnis zu reinigen bzw. zu desinfizieren, wobei die Reinigungstücher in einer bestimmten Reihenfolge anzuwenden sind. Durch die unterschiedliche Farbmarkierung der Verpackungen der drei Reinigungstücher wird die vorgesehene Reihenfolge der Verwendung der Reinigungstücher gemäß einem Ampelschema signalisiert. Vorteilhaft ist es hierbei, wenn dem Ampelschema folgend zunächst das rote Reinigungstuch 33, dann das gelbe Reinigungstuch 32 und abschließend das grüne Reinigungstuch 31 verwendet wird. Mit der Verwendung des grünen Reinigungstuches wird signalisiert, dass der Aufbereitungsvorgang dann abgeschlossen ist.

Die Farbmarkierungen auf den Verpackungen der Reinigungstücher 31, 32, 33 sind so ausgestaltet, dass diese vom Personal deutlich erkennbar sind, um ein Vertauschen der zu verwendenden Reinigungstücher weitestgehend auszuschließen. Die Verwendung von breiten, farbigen Streifen hat sich hierbei als vorteilhaft herausgestellt. Selbstverständlich können die Farbmarkierungen auch anders ausgestaltet sein, wobei lediglich sicherzustellen ist, dass die Farbmarkierungen gut erkennbar sind.

In einer alternativen Ausgestaltung der Erfindung können die Verpackungen der Reinigungstücher transparent ausgestaltet sein, wobei in diesem Fall die Reinigungstücher selbst farbig sein können. Beispielsweise kann für das erste zu verwendende Reinigungstuch ein rotes Reinigungstuch vorgesehen werden, welches mit der entsprechenden Reinigungslösung benetzt ist und in einer transparenten Verpackung verpackt ist.

Um sicherzustellen, dass vor Einbringen des Desinfektionstuches 10 in das Behältnis das Behältnis tatsächlich aufbereitet wird und die Aufbereitung des Behältnisses vorschriftsgemäß erfolgt, hat es sich als vorteilhaft erwiesen, wenn das Desinfektionstuch 10 zusammen mit dem Reinigungssystem als fertig konfektioniertes System zur Verfügung gestellt wird. Als besonders vorteilhaft hat es sich hierbei erwiesen, wenn die Reinigungstücher 31, 32, 33 an dem Desinfektionstuch, d.h. an der Schutzverpackung 20 des Desinfektionstuches 10 befestigt werden, von wo sie zum Zwecke der Reinigung bzw. Desinfektion des Behältnisses abgenommen werden können. Als besonders vorteilhaft hat sich hierbei herausgestellt, wenn die Reinigungstücher 31, 32, 33 so an der Schutzverpackung 20 angebracht sind, dass sie nur in einer vorbestimmten Reihenfolge von der Schutzverpackung 20 abgenommen werden können, d.h., dass vor dem Entfernen des gelben Reinigungstuches das rote Reinigungstuch entfernt werden muss und vor dem Entfernen des grünen Reinigungstuches das gelbe Reinigungstuch entfernt werden muss.

Um dies zu gewährleisten, ist es vorgesehen, die Reinigungstücher 31, 32, 33 mit einem Befestigungsmittel 40 an der Schutzverpackung 20 lösbar zu befestigen. Das Befestigungsmittel 40 kann hier ein Befestigungsband, insbesondere ein Klebeband, sein, mit dem die Reinigungstücher an der Schutzverpackung 20 gehalten werden. Das Befestigungsmittel 40 und die Reinigungstücher 31, 32, 33 werden dabei so relativ zueinander angeordnet, dass die Reinigungstücher zwischen der Schutzverpackung 20 und dem Befestigungsmittel 40 angeordnet sind und von dem Befestigungsmittel 40 an der Schutzverpackung 20 gehalten werden.

Das Befestigungsmittel 40 bzw. das Klebeband 40 ist mit seinen beiden Endabschnitten 46, 47 an der Schutzverpackung 20 befestigt, beispielsweise angeklebt, wobei zwischen diesen beiden Endabschnitten die Reinigungstücher 31, 32, 33 angeordnet sind.

Zum Lösen der Reinigungstücher von der Schutzverpackung 20 ist an dem Klebeband 40 im Bereich des ersten Endabschnittes 46 eine Perforation 45 vorgesehen. Das Klebeband 40 kann entlang dieser Perforation 45 getrennt werden, sodass das Klebeband 40 weggeklappt werden kann und dadurch die Reinigungstücher freigegeben werden. Die Reinigungstücher sind vorzugsweise so nebeneinander angeordnet, dass beim Wegklappen des Befestigungsmittels 40 zunächst das rote Reinigungstuch 31, dann das gelbe Reinigungstuch 32 und zum Schluss das grüne Reinigungstuch 33 freigegeben werden.

Das Befestigungsmittel bzw. das Befestigungsband 40 kann zudem an der den Reinigungstüchern zugewandten Oberfläche im Bereich der Reinigungstücher eine Klebeschicht aufweisen, sodass die Reinigungstücher beim Entfernen bzw. Wegklappen des Befestigungsbandes 40 an dem Befestigungsband 40 haften bleiben. Dadurch wird vermieden, dass sich die Reinigungstücher beim Wegklappen des Befestigungsbandes 40 selbstständig von der Schutzverpackung 20 lösen und dadurch die durch die Anordnung der Reinigungstücher an der Schutzverpackung vorgegebene Reihenfolge durcheinander gerät. In einer alternativen Ausgestaltung können auch die Reinigungstücher mit einem Klebemittel an der Schutzverpackung 20 befestigt sein, sodass diese auch nach dem Wegklappen des Befestigungsbandes 40 an der Schutzverpackung 20 haften bleiben.

Zur noch besseren Sicherstellung, dass vor Einbringen des Desinfektionstuches 10 in das Behältnis das Behältnis tatsächlich aufbereitet wird und die Aufbereitung des Behältnisses vorschriftsgemäß erfolgt, hat es sich als vorteilhaft erwiesen, wenn die Schutzverpackung 20 eine Sollbruchstelle 21 aufweist, entlang der die Schutzverpackung zur Entnahme des Desinfektionstuches geöffnet werden kann. Die Sollbruchstelle 21 verläuft hierbei so, dass sie von dem Befestigungsband 40 zumindest teilweise abgedeckt (gepunkteter Abschnitt) wird. Damit kann die Schutzverpackung 20 jedenfalls im Bereich des Befestigungsbandes 40 erst zerstört werden, nachdem das Befestigungsband abgenommen wurde und die Reinigungstücher zweckentsprechend verwendet wurden. Die Sollbruchstelle 21a kann auch quer zum Befestigungsband verlaufen.

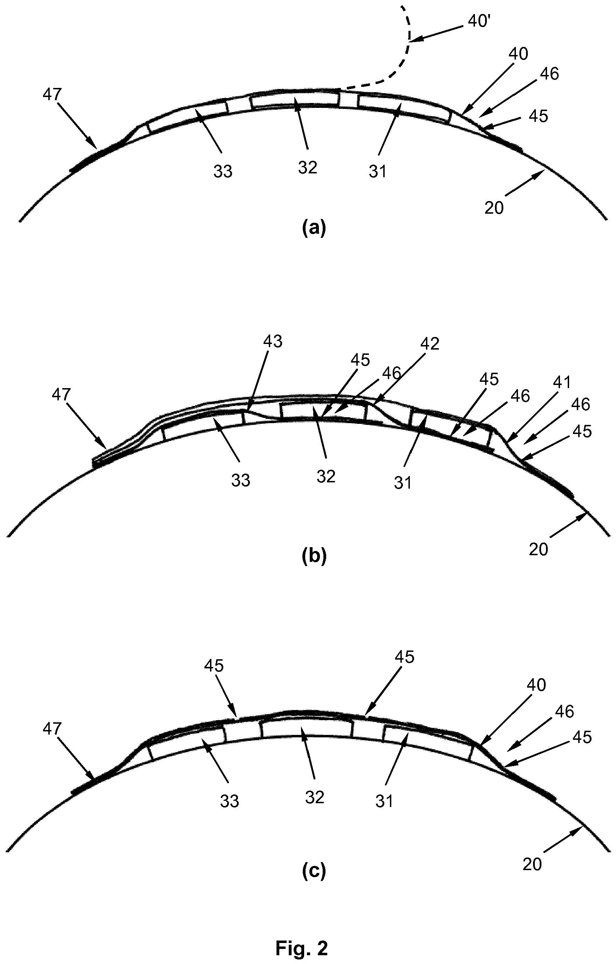

Fig. 2 zeigt drei verschiedene Ausgestaltungen eines Reinigungssystems, das an einer Schutzverpackung 20 eines Desinfektionstuches 10 angeordnet bzw. befestigt ist.

Die Abbildung (a) zeigt das Befestigungssystem aus Fig. 1 in einem Querschnitt.

An der Schutzverpackung 20 des Desinfektionstuches werden die drei Reinigungstücher 31, 32, 33 nebeneinander angeordnet, wobei die Reinigungstücher beabstandet zueinander angeordnet sein können. An der Schutzverpackung 20 wird ferner ein Befestigungsband 40 quer über die Reinigungstücher aufgebracht und an den Endabschnitten 46, 47 an der Schutzverpackung 20 befestigt, wobei zum Befestigen des Befestigungsbandes 40 an der Schutzverpackung 20 ein Klebemittel bzw. ein Klebstoff verwendet werden kann. Das Befestigungsband 40 ist hierbei derart straff angezogen, dass die Reinigungstücher 31, 32, 33 an die Schutzverpackung 20 gedrückt und damit sicher gehalten werden. Das Befestigungsband 40 kann in den Bereichen, in denen es an den Reinigungstüchern aufliegt, an der den Reinigungstüchern zugewandten Oberfläche eine Klebstoffschicht aufweisen, um ein noch besseres Befestigen der Reinigungstücher an der Schutzverpackung 20 zu ermöglichen.

An dem ersten Endabschnitt 46 weist das Befestigungsband 40 eine Perforation 45 auf, entlang der das Befestigungsband 40 durchtrennt werden kann, um das Befestigungsband 40 wegklappen zu können. Das Befestigungsband 40 ist vorzugsweise so ausgestaltet, dass ohne Verwendung von Hilfsmitteln, etwa eine Schere, das Befestigungsband nur entlang der Perforation 45 durchtrennt werden kann. Damit ist sichergestellt, dass die Reinigungstücher beim Wegklappen des Befestigungsbandes 40 auch tatsächlich in der vorgegebenen Reihenfolge zur Verwendung freigegeben werden.

Mit dem Bezugszeichen 40' ist ein teilweise weggeklapptes Befestigungsband gezeigt, wodurch das erste Reinigungstuch 31 zur Verwendung freigegeben ist. Bei einem weiteren Wegklappen des Befestigungsbandes 40 wird dann als nächstes das zweite Reinigungstuch 32 zur Verwendung freigegeben. Wird das Befestigungsband 40 schließlich vollständig weggeklappt, ist auch das dritte Reinigungstuch 33 zur Verwendung freigegeben.

Die Abbildung (b) zeigt eine alternative Ausgestaltung eines Reinigungssystems in einer Schnittansicht.

Im Unterschied zu dem in Abbildung (a) gezeigten Reinigungssystem sind bei dem in Abbildung (b) gezeigten Reinigungssystem drei Befestigungsbänder 41, 42, 43 vorgesehen, wobei mit jedem Befestigungsband jeweils ein Reinigungstuch an dem Desinfektionstuch bzw. an der Schutzverpackung 20 des Desinfektionstuches befestigt ist. Die Befestigungsbänder 41, 42, 43 sind dabei so relativ zueinander angeordnet, dass durch Lösen eines Befestigungsbandes jeweils nur ein Reinigungstuch zur Verwendung freigegeben wird, wobei ein weiteres Befestigungsband erst dann von der Schutzverpackung 20 gelöst werden kann, wenn bereits ein Reinigungstuch entnommen wurde.

So muss bei der in Abbildung (b) gezeigten Ausgestaltung zunächst das erste Befestigungsband 41 entlang einer Perforation 45 durchtrennt werden und anschließend weggeklappt werden, um das erste Reinigungstuch 31 zu entnehmen. Weil das erste Reinigungstuch 31 auf dem ersten Endabschnitt des zweiten Befestigungsbandes 42 aufliegt, kann auf dieses erst zugegriffen werden, wenn das erste Reinigungstuch entnommen wurde. Nach der Entnahme des ersten Reinigungstuches 31 kann dann das zweite Befestigungsband 42 entlang seiner Perforation 45 getrennt und dann weggeklappt werden, um das zweite Reinigungstuch 32 zur Verwendung freizugeben. Mit dem dritten Befestigungsband 43 und dem dritten Reinigungstuch 33 wird in entsprechender Weise verfahren.

Die gemäß Abbildung (b) vorgesehene Ausgestaltung eines Reinigungssystems erfordert zwar einen höheren Aufwand bei der Herstellung desselben, weist aber hinsichtlich der einzuhaltenden Reihenfolge der Reinigungstücher beim Aufbereiten der Behältnisse den Vorteil auf, dass ein unbeabsichtigtes Vertauschen der Reinigungstücher und damit die Verwendung der Reinigungstücher in einer falschen Reihenfolge noch besser vermieden wird.

Die Abbildung (c) zeigt eine noch weitere Ausgestaltung eines Reinigungssystems in einer Schnittansicht.

Das Reinigungssystem gemäß Abbildung (c) ist weitgehend identisch zu dem Reinigungssystem gemäß Abbildung (a). Der Unterschied zu dem Reinigungssystem gemäß Abbildung (a) besteht darin, dass das Befestigungsband 40 zusätzlich zu der im ersten Endabschnitt 46 vorgesehenen Perforierung zwei weitere Perforierungen 45 aufweist, die im Wesentlichen zwischen jeweils zwei Reinigungstüchern angeordnet sind.

Zwischen den Perforierungen und zwischen dem zweiten Endabschnitt 47 und der dem zweiten Endabschnitt benachbarten Perforierung 45 kann das Befestigungsband 40 an der den Reinigungstüchern zugewandten Oberfläche ein Klebemittel aufweisen, um die Reinigungstücher an dem Befestigungsband lösbar zu befestigen.

Wie bei der Ausgestaltung nach Abbildung (a) ist es auch hier vorgesehen, dass das Befestigungsband so ausgestaltet ist, dass es nur entlang der drei Perforierungen 45 ohne weitere Hilfsmittel durchtrennt werden kann. Das Befestigungsband 40 wird zunächst entlang der am ersten Endabschnitt 46 vorgesehenen Perforierung 45 durchtrennt und anschließend an der zwischen dem ersten Reinigungstuch 31 und dem zweiten Reinigungstuch 32 verlaufenden Perforierung. Damit kann das erste Reinigungstuch 31 samt dem herausgetrennten Abschnitt des Befestigungsbandes von der Schutzverpackung 20 abgenommen werden, wobei die übrigen Reinigungstücher 32, 33 weiterhin an der Schutzverpackung 20 verbleiben bzw. an dem restlichen Befestigungsband 40 haften bleiben. Mit dem zweiten bzw. dritten Reinigungstuch 32, 33 wird entsprechend verfahren.

Alle drei der in den Abbildungen (a) bis (c) gezeigten Ausgestaltungen eines Reinigungssystems gewährleisten, dass die Reinigungstücher in der für die Verwendung vorgesehenen Reihenfolge beim Lösen des Befestigungsbandes bzw. der Befestigungsbänder freigegeben werden.

Bei dem in Abbildung (a) bis Abbildung (c) gezeigten Ausgestaltung eines Reinigungssystems weist die Verpackung des ersten Reinigungstuches 31 eine rote Farbmarkierung, die Verpackung des zweiten Reinigungstuches 32 eine gelbe Farbmarkierung und die Verpackung des dritten Reinigungstuches 33 eine grüne Farbmarkierung auf, sodass bei Ablösen des Befestigungsbandes bzw. der Befestigungsbänder zunächst das rote Reinigungstuch, dann das gelbe Reinigungstuch und abschließend das grüne Reinigungstuch für die Verwendung freigegeben werden.

### Bezugszeichen:

- 10: Desinfektionstuch (z.B. perforierte Desinfektionstücher)
- 20: Schutzverpackung (z.B. Folie)
- 21: Sollbruchstelle (z.B. Perforation) der Schutzverpackung
- 21a: alternative Sollbruchstelle (z.B. Perforation) der Schutzverpackung
- 31: erstes Reinigungstuch
- 31a: erste Farbmarkierung (rot) auf der Verpackung des ersten Reinigungstuches
- 32: zweites Reinigungstuch
- 32a: zweite Farbmarkierung (gelb) auf der Verpackung des zweiten Reinigungstuches
- 33: drittes Reinigungstuch
- 33a: dritte Farbmarkierung (grün) auf der Verpackung des dritten Reinigungstuches
- 40: Befestigungsmittel (z.B. Befestigungsband aus Kunststoff oder Klebeband)
- 40': weggeklappter Abschnitt des Befestigungsmittels 40
- 41: erstes Befestigungsband
- 42: zweites Befestigungsband
- 43: drittes Befestigungsband
- 45: Perforation (im Bereich des ersten Endabschnittes 46 des Befestigungsbandes 40 bzw. zwischen in den Bereich zwischen jeweils zwei Reinigungstüchern)
- 46: erster Endabschnitt des Befestigungsbandes 40
- 47: zweiter Endabschnitt des Befestigungsbandes 40 (fest mit der Verpackung 20 verbunden)

## Patentansprüche

1. Desinfektionstuch (10), wobei
- das Desinfektionstuch vorgesehen ist, in ein Behältnis eingebracht zu werden, wobei das Behältnis vor Einbringen des Desinfektionstuches (10) einer Reinigung zu unterziehen ist,
- das Desinfektionstuch (10) in einer Schutzverpackung (20) verpackt ist, und
- das Desinfektionstuch (10) ohne die Schutzverpackung (20) in das Behältnis einbringbar ist,
**dadurch gekennzeichnet, dass**
- an der Schutzverpackung (20) ein Reinigungssystem angeordnet ist, zum Reinigen und/oder Desinfizieren des Behältnisses, wobei das Reinigungssystem zumindest ein erstes verpacktes Reinigungstuch (31), ein zweites verpacktes Reinigungstuch (32) und ein drittes verpacktes Reinigungstuch (33) umfasst, wobei die Verpackungen der Reinigungstücher (31; 32; 33) jeweils eine unterschiedliche Farbmarkierung aufweisen.

2. Desinfektionstuch nach dem vorhergehenden Anspruch, wobei die Reinigungstücher (31; 32; 33) mit einem Befestigungsmittel (40) lösbar an der Schutzverpackung (20) des Desinfektionstuches (10) befestigt sind.

3. Desinfektionstuch nach Anspruch 2, wobei das Befestigungsmittel (40) angepasst ist, beim Lösen des Befestigungsmittels (40) von der Schutzverpackung (20) des Desinfektionstuches (10) die Reinigungstücher (31; 32; 33) in einer vorbestimmten Reihenfolge zur Verwendung freizugeben, wobei das Befestigungsmittel (40) ein Befestigungsband umfasst, wobei zwischen der Schutzverpackung (20) und dem Befestigungsband (40) die Reinigungstücher (31; 32; 33) angeordnet sind, wobei das Befestigungsband (40) an einem ersten Endabschnitt (46) und an einem zweiten Endabschnitt (47) mit der Schutzverpackung (20) verbunden ist, wobei die Reinigungstücher (31; 32; 33) zwischen den beiden Endabschnitten (46; 47) angeordnet sind, wobei das Befestigungsband (40) im Bereich des ersten Endabschnittes (46) eine Perforation (45) aufweist, um das Befestigungsband zur Freigabe der Reinigungstücher (31; 32; 33) zu durchtrennen.

4. Desinfektionstuch nach einem der Ansprüche 2 bis 3, wobei das Befestigungsmittel (40) in den Bereichen zwischen jeweils zwei Reinigungstüchern (31; 32; 33) eine weitere Perforation (45) aufweist.

5. Desinfektionstuch nach Anspruch 2, wobei das Befestigungsmittel (40) ein erstes Befestigungsband (41), ein zweites Befestigungsband (42) und ein drittes Befestigungsband (43) umfasst, wobei mit jedem Befestigungsband (41; 42; 43) jeweils ein Reinigungstuch (31; 32; 33) lösbar an der Schutzverpackung (20) des Desinfektionstuches (10) befestigt ist, derart, dass eine Perforation (45) an einem ersten Endabschnitt (46) des zweiten und/oder dritten Befestigungsbandes (42; 43) erst dann freigegeben ist, wenn das erste Befestigungsband (41) von der Schutzverpackung (20) gelöst ist, und/oder wobei der erste Endabschnitt (46) des zweiten und/oder dritten Befestigungsbandes (42; 43) zwischen der Schutzverpackung (20) und dem ersten verpackten Reinigungstuch (31) und/oder dem zweiten verpackten Reinigungstuch (32) angeordnet ist.

6. Desinfektionstuch nach einem der Ansprüche 2 bis 5, wobei das Befestigungsband (40) zumindest in den Bereichen der Reinigungstücher (31; 32; 33) und an der den Reinigungstüchern zugewandten Oberfläche eine Klebeschicht aufweist.

7. Desinfektionstuch nach einem der vorhergehenden Ansprüche, wobei die Verpackung des ersten Reinigungstuches (31) eine erste, rote Farbmarkierung (31 a), die Verpackung des zweiten Reinigungstuches (32) eine zweite, gelbe Farbmarkierung (32a) und die Verpackung des dritten Reinigungstuches (33) eine dritte, grüne Farbmarkierung (33a) aufweisen.

8. Desinfektionstuch nach einem der vorhergehenden Ansprüche, wobei die Schutzverpackung (20) eine Sollbruchstelle (21; 21a) aufweist, entlang der die Schutzverpackung zur Entnahme des Desinfektionstuch (10) geöffnet werden kann und wobei die Sollbruchstelle (21; 21a) und die Befestigungsmittel (40) derart relativ zueinander angeordnet sind, dass das Befestigungsmittel (40) die Sollbruchstelle (21; 21 a) zumindest teilweise überdeckt.

9. System, umfassend einen Feuchttuchspender und ein Desinfektionstuch (10) nach einem der Ansprüche 1 bis 8.

## Claims

1. A disinfection tissue (10), wherein
- the disinfection tissue is provided for being inserted into a receptacle, said receptacle requiring cleaning prior to the insertion of said disinfection tissue(10),
- the disinfection tissue (10) is packaged in a protective packaging (20), and
- the disinfection tissue (10) may be inserted into the receptacle without the protective packaging (20),
**characterised in that**
- the protective packaging (20) has a cleaning system disposed thereon for cleaning and/or disinfecting the receptacle, said cleaning system comprising at least one first packaged cleaning tissue (31), one second packaged cleaning tissue (32), and one third packaged cleaning tissue (33), the packagings of the cleaning tissues (31; 32; 33) having each a different colour marking.

2. The disinfection tissue as claimed in the preceding claim, wherein the cleaning tissues (31; 32; 33) are detachably fixed to the protective packaging (20) of the disinfection tissue (10) via a fastening means (40).

3. The disinfection tissue as claimed in claim 2, wherein the fastening means (40) is adapted in such a manner that, as the fastening means (40) is being detached from the protective packaging (20) of the disinfection tissue (10), said cleaning tissues (31; 32; 33) are made accessible for use in a predetermined order, said fastening means (40) comprising a fastening tape, said cleaning tissues (31; 32; 33) being disposed between the protective packaging (20) and the fastening tissue (40), the fastening tape (40) being attached to the protective packaging (20) at a first end portion (46) and at a second end portion (47), the cleaning tissues (31; 32; 33) being disposed between said two end portions (46; 47), wherein the fastening tape (40) has a perforation (45) arranged in the region of the first end portion (46) permitting to sever the fastening tape so as to have access to the cleaning tissues (31; 32; 33).

4. The disinfection tissue as claimed in any one of claims 2 to 3, wherein the fastening means (40) has a further perforation (45) formed respectively in each region between two adjacent ones of the cleaning tissues (31; 32; 33).

5. The disinfection tissue as claimed in claim 2, wherein the fastening means (40) comprises a first fastening tape (41), a second fastening tape (42), and a third fastening tape (43), each fastening tape (41; 42; 43) detachably fastening a respective one of the cleaning tissues (31; 32; 33) to the protective packaging (20) of the disinfection tissue (10) in such a manner that the perforation (45) formed on a first end portion (46) of the second and/or the third fastening tape (42; 43) may only be accessed once the first fastening tape (41) has been detached from the protective packaging (20), and/or wherein the first end portion (46) of the second and/or third fastening tape (42; 43) is disposed between the protective packaging (20) and the first packaged cleaning tissue (31) and/or the second packaged cleaning tissue (32).

6. The disinfection tissue as claimed in any one of claims 2 to 5, wherein the fastening tape (40) has an adhesive layer formed at least in the regions of the cleaning tissues (31; 32; 33) and on its surface facing towards said cleaning tissues.

7. The disinfection tissue as claimed in any of the preceding claims, wherein the packaging of the first cleaning tissue (31) has a first, red colour marking (31 a), the packaging of the second cleaning tissue (32) has a second, yellow colour marking (32a), and the packaging of the third cleaning tissue (33) has a third, green colour marking (33a).

8. The disinfection tissue as claimed in any of the preceding claims, wherein the protective packaging (20) has a breaking point (21; 21a) along which the protective packaging may be opened for removal of the disinfection tissue (10), and wherein the breaking point (21; 21 a) and the fastening means (40) are disposed in such a manner relative to one another that the fastening means (40) at least partially covers the breaking point (21; 21 a).

9. A system comprising a wet tissue dispenser and a disinfection tissue (10) as claimed in any one of claims 1 to 8.

## Revendications

1. Lingette désinfectante (10), dans laquelle
- il est prévu que la lingette désinfectante soit introduite dans un contenant, le contenant devant être soumis à un nettoyage avant l'insertion de la lingette désinfectante (10),
- la lingette désinfectante (10) est conditionnée dans un emballage protecteur (20), et
- la lingette désinfectante (10) peut être insérée sans l'emballage protecteur (20) dans le contenant,
**caractérisée en ce qu'**
- un système de nettoyage est disposé sur l'emballage protecteur (20) en vue de nettoyer et/ou désinfecter le contenant, le système de nettoyage comprenant au moins une première lingette nettoyante emballée (31), une deuxième lingette nettoyante emballée (32) et une troisième lingette nettoyante emballée (33), les emballages des lingettes nettoyantes (31; 32; 33) présentant respectivement un marquage de couleur différente.

2. Lingette désinfectante selon la revendication précédente, dans laquelle les lingettes nettoyantes (31; 32; 33) sont fixées de manière amovible sur l'emballage protecteur (20) de la lingette désinfectante (10) grâce à un moyen de fixation (40).

3. Lingette désinfectante selon la revendication 2, dans laquelle le moyen de fixation (40) est conçu pour libérer les lingettes nettoyantes (31; 32; 33) dans un ordre prédéterminé en vue de leur utilisation lorsque ledit moyen de fixation (40) est détaché de l'emballage protecteur (20) de la lingette désinfectante (10), le moyen de fixation (40) comprenant un ruban de fixation, les lingettes nettoyantes (31; 32; 33) étant disposées entre l'emballage protecteur (20) et le ruban de fixation (40), le ruban de fixation (40) étant relié à l'emballage protecteur (20) au niveau d'une première partie d'extrémité (46) et d'une deuxième partie d'extrémité (47), les lingettes nettoyantes (31; 32; 33) étant disposées entre les deux parties d'extrémité (46; 47), le ruban de fixation (40) présentant dans la zone de la première partie d'extrémité (46) une perforation (45) permettant de détacher le ruban de fixation en vue de libérer les lingettes nettoyantes (31; 32; 33).

4. Lingette désinfectante selon l'une quelconque des revendications 2 à 3, dans laquelle le moyen de fixation (40) présente une autre perforation (45) dans les zones situées respectivement entre deux lingettes nettoyantes (31; 32; 33).

5. Lingette désinfectante selon la revendication 2, dans laquelle le moyen de fixation (40) comprend un premier ruban de fixation (41), un deuxième ruban de fixation (42) et un troisième ruban de fixation (43), dans laquelle chaque ruban de fixation (41; 42; 43) fixe respectivement de manière amovible une lingette nettoyante (31; 32; 33) sur l'emballage protecteur (20) de la lingette désinfectante (10) de telle sorte qu'une perforation (45) située sur une première partie d'extrémité (46) du deuxième et/ou troisième ruban de fixation (42; 43) n'est libérée que lorsque le premier ruban de fixation (41) a été détaché de l'emballage protecteur (20), et/ou dans laquelle la première partie d'extrémité (46) du deuxième et/ou troisième ruban de fixation (42; 43) est disposée entre l'emballage protecteur (20) et la première lingette nettoyante emballée (31) et/ou la deuxième lingette nettoyante emballée (32).

6. Lingette désinfectante selon l'une quelconque des revendications 2 à 5, dans laquelle le ruban de fixation (40) présente une couche de colle au moins dans les zones des lingettes nettoyantes (31; 32; 33), et ce sur la surface tournée vers les lingettes nettoyantes.

7. Lingette désinfectante selon l'une quelconque des revendications précédentes, dans laquelle l'emballage de la première lingette nettoyante (31) présente un premier marquage (31 a), de couleur rouge, l'emballage de la deuxième lingette nettoyante (32) un deuxième marquage (32a), de couleur jaune, et l'emballage de la troisième lingette nettoyante (33) un troisième marquage (33a), de couleur verte.

8. Lingette désinfectante selon l'une quelconque des revendications précédentes, dans laquelle l'emballage protecteur (20) présente une ligne destinée à la rupture (21; 21 a) le long de laquelle l'emballage protecteur peut être ouvert en vue de prélever la lingette désinfectante (10), la ligne destinée à la rupture (21; 21 a) et les moyens de fixation (40) étant disposés relativement l'un par rapport à l'autre de telle sorte que le moyen de fixation (40) recouvre au moins partiellement la ligne destinée à la rupture (21; 21 a).

9. Système comprenant un distributeur de lingettes humides et une lingette désinfectante (10) selon l'une quelconque des revendications 1 à 8.
